Europäisches Patentamt

European Patent Office

Office européen des brevets

(11) EP 1 281 385 A1

(19)

(12) **DEMANDE DE BREVET EUROPEEN**

(43) Date de publication:
05.02.2003 Bulletin 2003/06

(51) Int Cl.⁷: $A61K\ 7/032$

(21) Numéro de dépôt: 02291694.4

(22) Date de dépôt: 05.07.2002

(84) Etats contractants désignés:
AT BE BG CH CY CZ DE DK EE ES FI FR GB GR
IE IT LI LU MC NL PT SE SK TR
Etats d'extension désignés:
AL LT LV MK RO SI

(30) Priorité: 16.07.2001 FR 0109504

(71) Demandeur: L'OREAL
75008 Paris (FR)

(72) Inventeurs:
• Auguste, Frederic
94550 Chevilly-Larue (FR)
• Tournilhac, Florence
75011 Paris (FR)

(74) Mandataire: Boulard, Denis
L'OREAL-DPI
6 rue Bertrand Sincholle
92585 Clichy Cédex (FR)

(54) **Mascara comprenant des particules solides**

(57) L'invention a pour objet une composition de revêtement des fibres kératiniques comprenant, dans un milieu cosmétiquement acceptable comprenant au moins un solvant volatil, une fraction non volatile comprenant :

- a) un polymère apte à adhérer sur les matières kératiniques,
- b) des particules solides à 25 °C choisies parmi :

  - i) des particules solides d'un matériau cristallin ou semi-cristallin,
  - ii) des particules solides d'un matériau amorphe,
  - iii) des particules solides d'une cire ayant une dureté supérieure ou égale à 6,5 MPa
  - iv) et leurs mélanges,

les particules solides étant présentes dans la composition en une teneur telle que la fraction volumique des particules solides est inférieure ou égale à 50 % du volume total de ladite fraction non volatile.

La composition permet d'obtenir un bon recourbement des fibres kératiniques, notamment des cils.

EP 1 281 385 A1

**Description**

La présente invention a pour objet une composition cosmétique de revêtement des fibres kératiniques, notamment des cils ou des cheveux, comprenant des particules solides et un polymère adhérent, et son utilisation pour recourber les fibres kératiniques. La composition est destinée aux fibres kératiniques sensiblement longitudinales d'êtres humains comme les cils ou les cheveux ou bien encore aux faux-cils ou aux postiches comme les perruques. Plus spécialement, la composition est destinée au revêtement des cils.

La composition peut être une composition de maquillage, encore appelé mascara, une composition à appliquer sur un maquillage, dite encore top-coat, ou bien encore une composition de traitement des fibres kératiniques, notamment des cils. Plus spécialement, la composition est un mascara.

Le but de la présente invention est de proposer une composition de revêtement des cils conduisant après application, à un revêtement conférant un bon recourbement des cils.

Les inventeurs ont découvert qu'un tel revêtement des cils pouvait être obtenu en utilisant des particules solides particulières associées à un polymère adhérent.

De façon plus précise, l'invention a pour objet une composition de revêtement des fibres kératiniques, notamment des cils, comprenant, dans un milieu cosmétiquement acceptable comprenant au moins un solvant volatil, une fraction non volatile comprenant :

- a) un polymère apte à adhérer sur les matières kératiniques,
- b) des particules principales solides à 25 °C choisies parmi :

  - i) des premières particules solides comprenant un premier matériau cristallin ou semi-cristallin solide à 25 °C présentant une transition de phase de premier ordre, de fusion ou de combustion, supérieure à 100 °C,
  - ii) des deuxièmes particules solides comprenant un deuxième matériau amorphe ayant une température de transition vitreuse supérieure ou égale à 60 °C,
  - iii) des troisièmes particules solides comprenant un troisième matériau choisi parmi les cires ayant une dureté supérieure ou égale à 6,5 MPa,
  - iv) et leurs mélanges,

- c) et éventuellement des particules solides additionnelles différentes desdites particules solides principales, les particules solides additionnelles n'étant pas aptes à coalescer à une température inférieure ou égale à 40 °C,

les particules solides principales et, le cas échéant, les particules solides additionnelles étant présentes dans la composition en une teneur telle que la fraction volumique des particules solides principales et, le cas échéant, des particules solides additionnelles est supérieure ou égale à 1 % et inférieure à 50 % du volume total de ladite fraction non volatile,
et, le cas échéant, la fraction volumique des particules solides principales est supérieure ou égal à 50 % du volume total des particules solides principales et des particules solides additionnelles.

L'invention a également pour objet un procédé de maquillage ou de soin non thérapeutique des fibres kératiniques, notamment des cils, comprenant l'application sur les fibres kératiniques d'une composition telle que définie précédemment.

L'invention a aussi pour objet l'utilisation d'une composition telle que définie précédemment pour recourber les fibres kératiniques, notamment les cils.

L'invention a encore pour objet l'utilisation de particules principales solides à 25 °C choisies parmi

- i) des premières particules solides comprenant un premier matériau cristallin ou semi-cristallin solide à 25 °C présentant une transition de phase de premier ordre, de fusion ou de combustion, supérieure à 100 °C,
- ii) des deuxièmes particules solides comprenant un deuxième matériau amorphe ayant une température de transition vitreuse supérieure ou égale à 60 °C,
- iii) des troisièmes particules solides comprenant un troisième matériau choisi parmi les cires ayant une dureté supérieure ou égale à 6,5 MPa,
- iv) et leurs mélanges,

et éventuellement de particules solides additionnelles différentes desdites particules solides principales, les particules solides additionnelles n'étant pas aptes à coalescer à une température inférieure ou égale à 40 °C,
dans une composition de revêtement des fibres kératiniques, notamment un mascara, comprenant, dans un milieu cosmétiquement acceptable comprenant au moins un solvant volatil, une fraction non volatile comprenant un polymère apte à adhérer sur les matières kératiniques, lesdites particules solides principale et, éventuellement, lesdites particules solides additionnelles, les particules solides principales et, le cas échéant, les particules solides additionnelles étant présentes dans la composition en une teneur telle que la fraction volumique des particules solides principales et, le cas échéant, des particules solides additionnelles est supérieure ou égal à 1 % et inférieure à 50 % du volume total de ladite fraction non volatile,
et, le cas échéant, la fraction volumique des particules solides principales est supérieure ou égal à 50 % du volume total des particules solides principales et des particules solides additionnelles,

pour recourber les fibres kératiniques, notamment les cils.

On entend par particules solides des particules qui sont à l'état solide à 25 °C et à pression atmosphérique.

On entend par fraction non volatile de la composition l'ensemble des constituants présents dans la composition qui ne sont pas volatiles. On entend par composé volatile un composé qui, pris isolément, a une pression de vapeur non nulle, à température ambiante (25 °C) et pression atmosphérique, allant en particulier de $10^{-2}$ à 300 mm de Hg (1,33 Pa à 40.000 Pa) et de préférence supérieure à 0,3 mm de Hg (40 Pa).

La fraction non volatile de la composition correspond en fait au mélange des constituants restant sur les cils après le séchage complet du mascara appliqué sur les cils.

Pour obtenir un bon recourbement des cils, la composition selon l'invention comprend des particules solides, dites particules solides principales, choisies parmi les premières, deuxièmes, et troisièmes particules solides telles que définies précédemment, et leurs mélanges.

Les particules solides principales peuvent comprendre des particules solides, appelées premières particules solides, comprenant (en particulier formé de) un matériau, dit premier matériau, cristallin ou semi-cristallin solide à température ambiante (25 °C) présentant une transition de phase de premier ordre, de fusion ou de combustion, supérieure à 100 °C, de préférence supérieure à 120 °C, et mieux supérieure à 150 °C.

La température de fusion ou de combustion du premier matériau peut être mesurée selon la norme ASTM E794-98.

Par "matériau semi-cristallin", on entend au sens de l'invention, un matériau, notamment un polymère, comportant une partie cristallisable et une partie amorphe présentant une température de changement de phase réversible du premier ordre, en particulier de fusion (transition solide-liquide).

Avantageusement, le premier matériau cristallin ou semi cristallin présente une dureté Vickers supérieure ou égale à 10, notamment allant de 10 à 7500, de préférence supérieure ou égale à 200, notamment allant de 200 à 7500, et mieux supérieure ou égale à 400, notamment allant de 400 à 7500.

La dureté VICKERS (HV) est déterminée en appliquant sur le matériau un pénétromètre en forme de pyramide à base carrée, à l'aide d'une charge P. On mesure ensuite la dimension moyenne d'une diagonale de l'empreinte carrée obtenue avec le pénétromètre.

La dureté VICKERS (HV) est alors calculée par la relation :

$$HV = \frac{1854,4 \times P}{d^2}$$

d = diagonale moyenne en µm

P = charge appliquée en g

La mesure de la dureté VICKERS peut être effectuée à l'aide du microduromètre M 400 g 2 de la société LECO.

Le premier matériau desdites premières particules primaires peut être un matériau minéral qui peut être choisi parmi la silice, le verre, le diamant, le cuivre, le nitrure de bore, les céramiques, les micas, les oxydes métalliques, notamment les oxydes de fer comme l'oxyde de fer noir, l'oxyde de fer rouge, l'oxyde de fer jaune, les oxydes de titane, l'alumine, ou bien encore un polymère comme les polyamides par exemple le nylon, et leurs mélanges.

Les dites premières particules peuvent être des particules pleines, ou bien encore des particules creuses.

Selon un premier mode de réalisation de la composition selon l'invention, lesdites premières particules sont formées uniquement dudit premier matériau défini précédemment.

Selon un deuxième mode de réalisation de la composition selon l'invention, les premières particules solides comprennent au moins deux premiers matériaux distincts. C'est par exemple le cas des micas recouverts d'oxyde de titane ou d'oxyde de fer.

Selon un troisième mode de réalisation de la composition selon l'invention, lesdites premières particules solides comprennent au moins ledit premier matériau, et au moins un matériau additionnel, différent dudit premier matériau, ledit premier matériau formant la surface desdites premières particules. Pour ces particules solides, ledit premier matériau ayant les caractéristiques décrites précédemment se trouve à la surface desdites premières particules, ces dernières comprenant un matériau additionnel recouvert par le premier matériau.

Avantageusement, les dites premières particules peuvent avoir une taille moyenne allant de 5 nm à 50 µm, et de préférence de 20 nm à 50 µm.

Les particules solides principales peuvent comprendre des particules solides, appelées deuxièmes particules solides comprenant (en particulier formé de) un matériau, dit deuxième matériau, matériau amorphe, en particulier un polymère, ayant une température de transition vitreuse supérieure ou égale à 60 °C (notamment allant de 60 °C à 800 °C), avantageusement supérieure ou égale à 80 °C (notamment allant de 80 °C à 700 °C), et de préférence supérieure ou égale à 100 °C (notamment allant de 100 °C à 500 °C). La température de transition vitreuse peut être mesurée par DSC (Differential Scanning Calorimetry) selon la norme ASTM D3418-97.

Comme matériau amorphe, on peut utiliser un polymère non filmogène à une température inférieure ou égale à 40 °C ayant une température de transition vitreuse telle que décrite précédemment.

On entend par « polymère non filmogène à une température inférieure ou égale à 40 °C» un polymère qui n'est pas apte à former à lui seul, ou en présence d'agent auxiliaire de filmification, un film continu et adhérent sur un support, notamment sur les matières kératiniques, à une température inférieure ou égale à 40 °C.

On entend par agent auxiliaire de filmification les agents plastifiants et les agents de coalescence connus de l'homme du métier pour favoriser la filmification des polymères.

Comme polymère amorphe ayant une température de transition vitreuse supérieure ou égale à 60 °C, on peut utiliser des polymères radicalaires ou les polycondensats ayant la température de transition vitreuse définie.

Comme polymère radicalaire, on peut citer :

- les polymères d'éthylène, notamment de cycloéthylène, de naphtyléthylène ;
- les polymères de propylène, notamment d'hexafluoropropylène ;
- les polymères acryliques, notamment les polymères d'acide acrylique, d'acrylate de diméthyl-adamanthyl, de chloroacrylate ;
- les polymères d'acrylamide ;
- les polymères de (méth)acrylonitrile ;
- les polymères d'acétylstyrène, de carboxystyrène, de chlorométhylstyrène ;

Comme polycondensats, on peut citer les polycarbonates, les polyuréthanes, les polyesters, les polyamides, les polysulfones, les polysulfonamides, les carbohydrates comme l'amylose triacétate.

Les deuxièmes particules solides peuvent être des particules pleines ou des particules creuses.

Selon un premier mode de réalisation, les deuxièmes particules solides sont formées essentiellement dudit deuxième matériau amorphe décrit précédemment.

Selon un deuxième mode de réalisation, les deuxièmes particules solides comprennent au moins ledit deuxième matériau amorphe et au moins un matériau additionnel, différent du deuxième matériau amorphe, ledit deuxième matériau amorphe formant la surface, ou l'écorce, desdites deuxièmes particules solides et le matériau additionnel formant l'intérieur, ou le coeur, desdites deuxièmes particules solides.

Le matériau additionnel peut être par exemple un polymère additionnel ayant une température de transition vitreuse inférieure à 60 °C, et de préférence inférieure à 45 °C.

Ainsi, les deuxièmes particules solides peuvent être par exemple des particules coreshell de polymères, comprenant une partie externe (c'est-à-dire une écorce) formée du premier matériau amorphe ayant une température de transition vitreuse supérieure ou égale à 60 °C et comprenant une partie interne (c'est-à-dire un coeur) ayant une température de transition vitreuse inférieure à 60 °C.

Avantageusement, la teneur du premier matériau amorphe dans les deuxièmes particules solides est telle que la fraction volumique du premier matériau est supérieure ou égale à 10 %, et de préférence supérieure ou égale à 30 %, en volume du volume total des deuxièmes particules solides.

Les deuxièmes particules solides peuvent avoir une taille moyenne allant de 10 nm à 50 μm, et de préférence allant de 20 nm à 1 μm.

Comme deuxièmes particules solides, on peut utiliser les dispersions aqueuses de polymère non filmogène vendues sous les dénominations « JONCRYL® SCX 8082 » , « JONCRYL® 90 » par la société JOHNSON POLYMER, « NEOCRYL® XK 52 » par la société AVECIA RESINS, « RHODOPAS® 5051 » par la société RHODIA CHIMIE.

Les particules solides principales peuvent comprendre des particules solides, appelées troisièmes particules solides, comprenant (en particulier formé de) une cire, appelée cire dure, ayant une dureté supérieure ou égale à 6,5 MPa.

Par "cire", on entend au sens de la présente invention, un composé gras lipophile, solide à température ambiante (25 °C) et pression atmosphérique (760 mm de Hg, soit $10^5$ Pa), à changement d'état solide/ liquide réversible, ayant une température de fusion de fusion allant de 30 °C à 99 °C, et mieux allant de 45 °C à 99 °C.

En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les valeurs de point de fusion correspondent, selon l'invention, au pic de fusion mesurée à l'aide d'un calorimètre à balayage différentiel (D.S.C.), par exemple le calorimètre vendu sous la dénomination DSC 30 par la société METLER , avec une montée en température de 5 ou 10 °C par minute.

La cire dure peut avoir un point de fusion allant de 30 °C à 99°C, et notamment allant de 40 °C à 99 °C.

Avantageusement, la cire dure a un point de fusion supérieur ou égal à 30 °C et inférieur à 77 °C, en particulier supérieur ou égal à 30 °C et inférieur à 60 °C, notamment allant de 30 °C à 59 °C, de préférence allant de 35 °C à 59 °C, et mieux allant de 40 °C à 50 °C.

De préférence, la cire peut avoir une dureté allant de 6,5 MPa à 20 MPa, notamment allant de 9,7 MPa à 20 MPa, et en particulier allant de 9,7 MPa à 15 MPa. Avantageusement, la cire peut avoir une dureté supérieure à 10 MPa, notamment allant de 10 à 20 MPa, et mieux allant de 10 à 12 MPa.

Selon la présente demande, la dureté de la cire est déterminée par la mesure de la force en compression mesurée à 20 °C à l'aide du texturomètre vendu sous la dénomination TA-XT2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm. Pour effectuer la mesure de dureté, la cire est fondue à une température égale au point de fusion de la cire + 20 °C. La cire fondue est coulée dans un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté. La valeur de la dureté est la force de compression mesurée divisée par la surface du cylindre du texturomètre en contact avec la cire.

Comme cire répondant aux critères définis précédemment, on peut utiliser la cire de Candellila, la cire de jojoba hydrogénée, la cire de sumac, la cérésine, le stéarate d'octacosanyle, le stéarate de tétracontanyle, la cire de Shellac, le fumarate de béhényle, le tétrastéarate de di-(triméthylol-1,1,1 propane) vendu sous la dénomination « HEST 2T-4S » par la société HETERENE, le tétrabéhénate de di-(triméthylol-1,1,1 propane) vendue sous la dénomination HEST 2T-4B par la société HETERENE, les ozokerites comme celle vendue sous la dénomination « OZOKERITE WAX SP 1020 P » par la société STRAHL & PITSCH

On peut également utiliser la cire obtenue par hydrogénation d'huile d'olive estérifiée avec l'alcool stéarylique vendue sous la dénomination « PHYTOWAX Olive 18 L 57 » ou bien encore les cires obtenues par hydrogénation d'huile de ricin estérifiée avec l'alcool cétylique vendus sous la dénomination « PHYTOWAX ricin 16L64 et 22L73 », par la société SOPHIM. De telles cires sont décrites dans la demande FR-A- 2792190.

Avantageusement, la cire dure est choisie parmi la cire d'olive obtenue par hydrogénation d'huile d'olive estérifiée avec l'alcool stéarylique vendue sous la dénomination PHYTOWAX Olive 18 L 57 de la société SOPHIM et le tétrastéarate de di-(triméthylol-1,1,1 propane).

Avantageusement, les troisièmes particules de cire dure peuvent avoir une taille moyenne allant de 50 nm à 50 μm, et de préférence allant de 50 nm à 10 μm.

Selon un premier mode de réalisation de la composition selon l'invention, les particules solides principales sont constituées des premières ou deuxièmes ou troisièmes particules solides décrites précédemment.

Selon un deuxième mode de réalisation de la composition selon l'invention, les particules solides principales sont constituées du mélange des premières et des deuxièmes particules solides, ou des deuxièmes et troisièmes particules solides, ou des premières et troisièmes particules solides décrites précédemment.

Selon un troisième mode de réalisation de la composition selon l'invention, les particules solides principales sont constituées du mélange des premières, deuxièmes et troisièmes particules solides décrites précédemment.

La composition selon l'invention peut comprendre, en plus des premières particules solides principales décrites précédemment, d'autres particules solides, appelées particules solides additionnelles, différentes des particules solides principales.

Ces particules solides additionnelles correspondent aux particules solides à 25 °C de tout matériau, différent des particules solides principales, restant sous forme de particules individualisées, ou éventuellement collées mais qui conservent dans ce cas leur état de particule individuelle (ces particules collées ne sont pas coalescées à une température inférieure ou égale à 40 °C).

Toutes les constituants présents dans la composition selon l'invention se trouvant à l'état de particules solides à 25 °C et qui ne coalescent pas à une température inférieure ou égale à 40 °C à eux seuls ou en présence des autres constituants présents dans la composition sont considérées comme étant soit des particules solides principales ou soit des particules solides additionnelles selon les définitions décrites précédemment.

Ainsi, par exemple, les particules solides additionnelles peuvent être en un matériau choisi parmi les cires différentes de la cire dure des troisièmes particules décrite précédemment, les charges, les polymères différents du matériau amorphe présent dans les deuxièmes particules solides décrites précédemment.
Les additifs décrits ci-après, lorsqu'ils sont sous la forme de particules solides à 25 °C, sont considérés comme étant soit des particules solides principales, soit des particules solides additionnelles telles que décrites précédemment lorsque ces additifs possèdent les caractéristiques correspondantes définies précédemment.

En particulier, le polymère adhérent présent dans la composition selon l'invention peut être sous la forme de particules solides. Dans ce cas, ces particules sont considérées comme étant des particules solides telles que définies précédemment si ce polymère possède les caractéristiques définies précédemment.

Dans la composition selon l'invention, les particules solides principales et, le cas échéant, les particules solides additionnelles sont présentes en une teneur telle que la fraction volumique des particules solides principales et, le cas échéant, des particules solides additionnelles est supérieure ou égale à 1 % et inférieure à 50 %, de préférence est supérieure ou égale à 5 % et inférieure à 50 %, du volume total de la fraction non volatile de la composition, ce qui signifie que le volume total de toutes les premières particules et, le cas échéant, des deuxièmes particules représente au moins 1 % mais moins de 50 % (notamment va de 1 % à 49 %, et de préférence va de 5 % à 49 %) du volume total de la fraction non volatile de la composition.

On entend par « fraction volumique des particules solides principales et, le cas échéant, des particules solides additionnelles» le pourcentage du volume total de toutes les particules solides principales et, le cas échéant, de toutes les particules solides additionnelles présent dans la fraction non volatile de la composition, par rapport au volume total de tous les composés de la fraction non volatile de la composition.

Avantageusement, ladite fraction volumique des particules solides principales et, le cas échéant, des particules solides additionnelles est supérieure ou égale à 1 % et inférieure ou égale à 40 % (notamment va de 5 % à 40 %), et de préférence supérieure ou égale à 1 % et inférieure ou égale à 30 % (notamment va de 10 % à 30 %) du volume total de la fraction non volatile de la composition.

La fraction volumique (FV) de particules solides présente dans la fraction non volatile de la composition est égale au pourcentage du volume total V desdites particules divisé par le volume total V' de la fraction non volatile de la composition.

Le volume V de particules solides est égal à la masse m desdites particules solides dans la composition divisé par la masse volumique Mv des particules. La masse volumique est calculée selon la méthode décrite ci-après.

Fraction volumique : $FV = 100 \times V / V'$ et $V = m / Mv$

Le volume total V' de la fraction non volatile de la composition est calculé en additionnant le volume de chaque constituant non volatil présent dans la composition.

Avantageusement, lorsque la composition comprend des particules solides additionnelles telles que définies précédemment, les particules solides principales sont présentes dans la composition en une teneur telle que la fraction volumique des particules solides principales est supérieure ou égale à 50 %, notamment va de 50 % à 99 %, du volume total des particules solides principales et des particules solides additionnelles, de préférence est supérieure ou égale à 60 %, notamment va de 60 % à 99 %, et mieux est supérieure ou égale à 70 %, notamment va de 70 % à 99 %.

Le solvant volatil présent dans la composition selon l'invention peut être choisi parmi l'eau, les solvants organiques volatils et les huiles volatiles définis ci-après, et leurs mélanges.

Dans la présente demande, on entend par « polymère apte à adhérer sur les matières kératiniques », appelé par la suite polymère adhérent, un polymère apte à rester fixé sur les matières kératiniques telles que les fibres kératiniques comme les cils , les cheveux, ou la peau, et de préférence sur les cils, lors du contact du polymère avec lesdites matières kératiniques. Un tel polymère adhérent a d'ailleurs une bonne aptitude à former un dépôt sur les matières kératiniques et reste fixé sur ces dernières.

Avantageusement, le polymère adhérent peut être un polymère filmogène à une température inférieure ou égale à 40 °C. Dans la présente demande, on entend par "polymère filmogène", un polymère apte à former à lui seul ou en présence d'un agent auxiliaire de filmification, un dépôt, notamment un film, continu et adhérent sur un support, notamment sur les matières kératiniques.

Le polymère adhérent présent dans la composition selon l'invention peut être un polymère solubilisé ou dispersé sous forme de particules solides dans une phase aqueuse de la composition ou bien encore solubilisé ou dispersé sous forme de particules solides dans une phase grasse liquide. La composition peut comprendre un mélange de ces polymères. Lorsque le polymère adhérent se présente sous forme de particules solides, ces particules peuvent présenter une taille moyenne de particules allant de 5 nm à 10 μm, notamment allant de 5 nm à 5 μm, avantageusement allant de 5 nm à 600 nm, et de préférence allant de 20 nm à 300 nm.

Le polymère adhérent peut être présent dans la composition selon l'invention en une teneur en matières sèches allant de 0,1 % à 50 % en poids par rapport au poids total de la composition, de préférence de 0,5 % à 40 % en poids, et mieux de 1 % à 30 % en poids.

Parmi les polymères adhérents utilisables dans la composition de la présente invention, on peut citer les polymères synthétiques, de type radicalaire ou de type polycondensat, les polymères d'origine naturelle, et leurs mélanges.

Par polymère radicalaire, on entend un polymère obtenu par polymérisation de monomères à insaturation notamment éthylénique, chaque monomère étant susceptible de s'homopolymériser (à l'inverse des polycondensats).

Les polymères de type radicalaire peuvent être notamment des polymères, ou des copolymères, vinyliques, notamment des polymères acryliques.

Les polymères vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique.

Les esters de monomères acides sont avantageusement choisis parmi les esters de l'acide (méth) acrylique (encore appelé les (méth)acrylates), notamment des (méth)acrylates d'alkyle, en particulier d'alkyle en $C_1$-$C_{30}$, de préférence en $C_1$-$C_{20}$, des (méth)acrylates d'aryle, en particulier d'aryle en $C_6$-$C_{10}$, des (méth) acrylates d'hydroxyalkyle, en particulier d'hydroxyalkyle en $C_2$-$C_6$.

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alk-yl en $C_2$-$C_{12}$. Parmi les N-alkyl (méth)acrylamides, on peut citer le N-éthyl acrylamide, le N-t-butyl acrylamide, le N-t-octyl acrylamide et le N-undécylacrylamide.

Les polymères vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle.

Comme monomères styrèniques, on peut citer le styrène et l'alpha-méthyl styrène.

Il est possible d'utiliser tout monomère connu de l'homme du métier entrant dans les catégories de monomères acryliques et vinyliques (y compris les monomères modifiés par une chaîne siliconée).

Parmi les polycondensats, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les polyuréthanes-polyvinylpirrolidones, les polyester-polyuréthanes, les polyéther-polyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.

L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexa-nedicarboxylique, l'acide isophtalique, l'acide téréphtalique, l'acide 2,5-norborane dicarboxylique, l'acide diglycolique, l'acide thiodipropionique, l'acide 2,5-naphtalènedicarboxylique, l'acide 2,6-naphtalè-nedicarboxylique. Ces monomères acide dicarboxyli-que peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol.

Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement -SO$_3$M, avec M représentant un atome d'hydrogène, un ion ammonium $NH_4^+$ ou un ion alcalin, alcalino-terreux ou métallique, comme par exemple un ion $Na^+$, $Li^+$, $K^+$, $Mg^{2+}$, $Ca^{2+}$, $Cu^{2+}$, $Fe^{2+}$, $Fe^{3+}$. On peut utiliser notam-ment un monomère aromatique bifonctionnel compor-tant un tel groupement -SO$_3$M.

Le noyau aromatique du monomère aromatique bifonctionnel portant en outre un groupement -SO$_3$M tel que décrit ci-dessus peut être choisi par exemple parmi les noyaux benzène, naphtalène, an-thracène, diphényl, oxydiphényl, sulfonyldiphényl, mé-thylènediphényl. On peut citer comme exemple de monomère aromatique bifonctionnel portant en outre un groupement -SO$_3$M: l'acide sulfoisophtalique, l'acide sulfotéréphtalique, l'acide sulfophtalique, l'acide 4-sulfonaphtalène-2,7-dicarboxylique.

On préfère utiliser des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique. De tels polymères sont vendus par exemple sous le nom de marque Eastman AQ® par la société Eastman Chemical Products.

Les polymères d'origine naturelle, éventuellement modifiés, peuvent être choisis parmi la résine shellac, la gomme de sandaraque, les dammars, les élémis, les copals, les polymères cellulosiques, et leurs mélanges.

Selon un premier mode de réalisation de la composition selon l'invention, le polymère adhérent peut être présent sous la forme de particules solides en dispersion aqueuse, connue généralement sous le nom de latex ou pseudolatex. Les techniques de préparation de ces dispersions sont bien connues de l'homme du métier.

Comme dispersion aqueuse de polymère adhérent, on peut utiliser les dispersions acryliques vendues sous les dénominations NEOCRYL XK-90®, NEOCRYL A-1070®, NEOCRYL A-1090®, NEOCRYL BT-62®, NEOCRYL A-1079®, NEOCRYL A-523® par la société AVECIA-NEORESINS, DOW LATEX 432® par la société DOW CHEMICAL, DAITOSOL 5000 AD® par la société DAITO KASEY KOGYO; ou bien encore les dispersions aqueuses de polyuréthane vendues sous les dénominations NEOREZ R-981®, NEOREZ R-974® par la société AVECIA-NEORESINS, les AVALURE UR-405®, AVALURE UR-410®, AVALURE UR-425 ®, AVALURE UR-450®, SANCURE 875®, SANCURE 861®, SANCURE 878®, SANCURE 2060® par la société GOODRICH, IMPRANIL 85® par la société BAYER, AQUAMERE H-1511® par la société HYDROMER.

Comme dispersion aqueuse de polymère adhérent, on peut également utiliser les dispersions de polymères résultant de la polymérisation radicalaire d'un ou plusieurs monomères radicalaires à l'intérieur eVou partiellement en surface, de particules préexistantes d'au moins un polymère choisi dans le groupe constitué par les polyuréthanes, les polyurées, les polyesters, les polyesteramides et/ou les alkydes. Ces polymères sont généralement appelés polymères hybrides.

Selon une deuxième variante de réalisation de la composition selon l'invention, le polymère adhérent peut être un polymère hydrosoluble et est donc présent dans la phase aqueuse de la composition sous forme solubilisée. Comme exemples de polymères filmogènes hydrosolubles, on peut citer

- les protéines comme les protéines d'origine végétale telles que les protéines de blé, de soja ; les protéines d'origine animale tels que les kératines, par exemples les hydrolysats de kératine et les kératines sulfoniques ;
- les polymères de chitine ou de chitosane anioniques, cationiques, amphotères ou non-ioniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;

- les polymères d'origine naturelle, éventuellement modifiés, tels que :

  - les gommes arabiques, la gomme de guar, les dérivés du xanthane, la gomme de karaya ;
  - les alginates et les carraghénanes ;
  - les glycoaminoglycanes, l'acide hyaluronique et ses dérivés ;
  - la résine shellac, la gomme de sandaraque, les dammars, les élémis, les copals ;
  - l'acide désoxyribonucléïque ;
  - les muccopolysaccharides tels que l'acide hyaluronique, les chondroïtines sulfate, et leurs mélanges.

Selon une autre variante de réalisation de la composition selon l'invention, le polymère adhérent peut être présent dans une phase grasse liquide comprenant des huiles ou solvants organiques tels que ceux décrits précédemment. Par "phase grasse liquide", on entend, au sens de l'invention, une phase grasse liquide à température ambiante (25°C) et pression atmosphérique (760 mm de Hg, soit $10^5$ Pa), composée d'un ou plusieurs corps gras liquides à température ambiante, appelés aussi huiles, généralement compatibles entre eux.

De préférence, la phase grasse liquide comprend une huile volatile, éventuellement en mélange avec une huile non volatile, les huiles pouvant être choisies parmi les huiles citées ci-après.

Selon un troisième mode de réalisation de la composition selon l'invention, le polymère adhérent peut être présent sous forme de particules, stabilisées en surface, dispersées dans la phase grasse liquide.

La dispersion de particules de polymère stabilisées en surface peut être fabriquée comme décrit dans le document EP-A-749747.

Les particules de polymère sont stabilisées en surface grâce à un stabilisant qui peut être un polymère séquencé, un polymère greffé, et/ou un polymère statistique, seul ou en mélange.

Des dispersions de polymère filmogène dans la phase grasse liquide, en présence d'agent stabilisants, sont notamment décrites dans les documents EP-A-749746, EP-A-923928, EP-A-930060 dont le contenu est incorporé à titre de référence dans la présente demande.

La taille des particules de polymères en dispersion soit dans la phase aqueuse, soit dans la phase grasse liquide, peut aller de 5 nm à 600 nm, et de préférence de 20 nm à 300 nm.

Selon un quatrième mode de réalisation de la composition selon l'invention, le polymère adhérent peut être solubilisé dans la phase grasse liquide, on dit alors que le polymère filmogène est un polymère liposoluble.

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester ) et d'au moins un autre monomère qui peut être un ester vinylique (différent de l'ester vinylique déjà présent), une α-oléfine (ayant de 8 à 28 atomes de carbone), un alkyivinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvlnyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères liposolubles, on peut également citer les homopolymères liposolubles, et en particulier ceux résultant de l'homopolymérisation d'esters vinyliques ayant de 9 à 22 atomes de carbone ou d'acrylates ou de méthacrylates d'alkyle, les radicaux alkyles ayant de 10 à 20 atomes de carbone.

De tels homopolymères liposolubles peuvent être choisis parmi le polystéarate de vinyle, le polystéarate de vinyle réticulé à l'aide de divinylbenzène, de diallyléther ou de phtalate de diallyle, le poly(méth)acrylate de stéaryle, le polylaurate de vinyle, le poly(méth)acrylate de lauryle, ces poly(méth)acrylates pouvant être réticulés à l'aide de diméthacrylate de l'éthylène glycol ou de tétraéthylène glycol.

Les copolymères et homopolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en $C_2$-$C_{20}$, comme le polybutène, les alkylcelluloses avec un radical alkyle linéaire ou ramifié, saturé ou non en $C_1$ à $C_8$ comme l'éthylcellulose et la propylcellulose, les copolymères de la vinylpyrolidone (VP) et notamment les copolymères de la vinylpyrrolidone et d'alcène en $C_2$ à $C_{40}$ et mieux en $C_3$ à $C_{20}$. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

La composition selon l'invention peut comprendre un agent auxiliaire de filmification favorisant la formation d'un film avec le polymère filmogène. Un tel agent de filmification peut être choisi parmi tous les composés connus de l'homme du métier comme étant susceptibles de remplir la fonction recherchée, et notamment être choisi parmi les agents plastifiants et les agents de coalescence.

Selon un mode de réalisation préféré de la composition selon l'invention, le polymère adhérent peut être un polymère apte à former un dépôt, notamment un film, produisant à une concentration de 7 % dans l'eau, une rétraction du stratum corneum isolé de plus de 1 % à 30°C sous une humidité relative de 40 %, de préférence de plus de 1,2 %, et mieux de plus de 1,5 %. Cette rétraction est mesurée à l'aide d'un extensiomètre, selon la méthode décrite ci-après.

Selon un premier mode de réalisation de la composition selon l'invention, la composition peut comprendre un milieu aqueux, constituant une phase aqueuse, qui peut être la phase continue de la composition.

La phase aqueuse peut être constituée essentiellement d'eau ; elle peut également comprendre un mélange d'eau et de solvant miscible à l'eau (solvant apte à former avec l'eau un mélange homogène et transparent à l'oeil à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en $C_3$-$C_4$, les aldéhydes en $C_2$-$C_4$.

La phase aqueuse (eau et éventuellement le solvant organique miscible à l'eau) peut être présente, en une teneur allant de 1 % à 95 % en poids, par rapport au poids total de la composition, de préférence de 3 % à 80 % en poids, et mieux de 5 % à 60 % en poids.

Selon un deuxième mode de réalisation de la composition selon l'invention, la composition peut comprendre au moins une huile ou solvant organique volatile qui peut notamment former une phase grasse, et en particulier une phase grasse continue. La composition peut être une composition anhydre.

Par " huile ou solvant organique volatile", on entend au sens de l'invention des solvants organiques et des huiles cosmétiques volatiles, liquides à température ambiante, ayant une pression de vapeur non nulle, à température ambiante et pression atmosphérique, allant en particulier de $10^{-2}$ à 300 mm de Hg (1,33 Pa à 40.000 Pa) et de préférence supérieure à 0,3 mm de Hg (30 Pa). Par "huile non volatile", on entend une huile restant sur la peau à température ambiante et pression atmosphérique au moins plusieurs heures et ayant notamment une pression de vapeur inférieure à $10^{-2}$ mm de Hg (1,33 Pa).

Ces huiles peuvent être des huiles hydrocarbonées, des huiles siliconées, des huiles fluorées, ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en $C_8$-$C_{16}$ comme les isoalcanes en $C_8$-$C_{16}$ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars' ou de Permetyls, les esters ramifiés en $C_8$-$C_{16}$ le néopentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées. De préférence, le solvant volatil est choisi parmi les huiles volatiles hydrocarbonées ayant de 8 à 16 atomes de carbone et leurs mélanges.

Comme huiles volatiles, on peut aussi utiliser les silicones volatiles, comme par exemple les huiles de silicones linéaires ou cycliques volatiles, notamment celles ayant une viscosité ≤ 8 centistokes (8 $10^{-6}$ m²/s), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile peut être présente dans la composition selon l'invention en une teneur allant de 0,1 % à 98 % en poids, par rapport au poids total de la composition, de préférence de 1 % à 65 % en poids.

La composition peut également comprendre au moins une huile non volatile, et notamment choisie parmi les huiles hydrocarbonées et/ou siliconées et/ou fluorées non volatiles.

Comme huile hydrocarbonée non volatile, on peut notamment citer :

- les huiles hydrocarbonées d'origine végétale telles que les triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_5 + R_6$ soit ≥ 10, comme par exemple l'huile de Purcellin (octanoate de cétostéaryle), le myristate d'isopropyle, le palmitate d'isopropyle, le benzoate d'alcool en $C_{12}$ à $C_{15}$, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;

et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Les huiles fluorées utilisables dans l'invention sont notamment des huiles fluorosiliconées, des polyéthers fluorés, des silicones fluorées telles que décrit dans le document EP-A-847752.

Les huiles non volatiles peuvent être présentes dans la composition selon l'invention en une teneur allant de 0,1 % à 50 % en poids, de préférence de 0,1 % à 30 % en poids, par rapport au poids total de la composition, et mieux de 0,1 % à 20 % en poids.

La composition selon l'invention peut comprendre en outre au moins une cire additionnelle différente de la cire des troisièmes particules solides décrites précédemment.

Comme cire additionnelle, on peut notamment citer la cire d'abeilles, la cire de lanoline, et les cires d'insectes de Chine; la cire de riz, la cire de Carnauba, certaines cires microcristallines, les cires de paraffine, certaines ozokérites, certaines cires de polyéthylène, certaines cires obtenues par la synthèse de Fisher-Tropsch.

On peut aussi citer les cires obtenues par hydrogénation catalytique d'huiles animales ou végétales ayant des chaînes grasses, linéaires ou ramifiées, en C8-C32. Parmi celles-ci, on peut notamment citer l'huile de tournesol hydrogénée, l'huile de ricin hydrogénée, l'huile de coprah hydrogénée et l'huile de lanoline hydrogénée.

On peut encore citer les cires de silicone ou les cires fluorées.

La cire additionnelle présente dans la composition peut être dispersée sous forme de particules, notamment dans le milieu aqueux de la composition. Ces particules peuvent avoir une taille moyenne allant de 50 nm à 50 $\mu$m, et de préférence allant de 50 nm à 10 $\mu$m.

En particulier, la cire additionnelle est généralement présente dans la composition selon l'invention sous forme de particules solides et font donc parties des particules solides additionnelles définies précédemment.

La cire additionnelle peut être présente dans la composition selon l'invention en une teneur allant de 0,1 % à 50 % en poids, par rapport au poids total de la composition, de préférence de 0,5 % à 30 % en poids, et mieux de 1 % à 20 % en poids.

La composition selon l'invention peut contenir des agents tensioactifs émulsionnants présents notamment en une proportion allant de 2 à 30 % en poids par rapport au poids total de la composition, et mieux de 5 % à 15 %. Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans la composition selon l'invention sont choisis :

- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de $C_1$-$C_6$ alkyl glucose polyoxyéthylénés, et leurs mélanges.

- parmi les tensioactifs anioniques : les acides gras en $C_{16}$-$C_{30}$ neutralisés par les amines, l'ammoniaque ou les sels alcalins, et leurs mélanges ; le copolymère acide acrylique /itaconate de monostéaryleoxyéthyléné (20 OE) en dispersion aqueuse à 30 % en poids vendu sous la dénomination « STRUCTURE 2001 » par la société National Starch, le copolymère acide acrylique/itaconate de monocétyle éthoxylé (20 OE) en dispersion aqueuse à 30 % vendu sous la dénomination « STRUCTURE 3001 » par la société National Starch.

On utilise de préférence des tensioactifs permettant l'obtention d'émulsion huile-dans-eau ou cire-dans-eau.

La composition selon l'invention peut également comprendre une matière colorante comme les matières colorantes pulvérulentes, les colorants liposolubles, les colorants hydrosolubles. Cette matière colorante peut être présente en une teneur allant de 0,01 % à 50 % en poids, par rapport au poids total de la composition, de préférence allant de 0,01 % à 30 % en poids.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C orange 5, le jaune quinoléine, le rocou. Les colorants hydrosolubles sont par exemple le jus de betterave, le bleu de méthylène.

La composition de l'invention peut comprendre, en outre, tout additif usuellement utilisé en cosmétique tels que les antioxydants, les charges, les conservateurs, les parfums, les neutralisants, les épaississants, les plastifiants, les agents de coalescence, les actifs cosmétiques ou dermatologiques comme par exemple des émollients, des hydratants, des vitamines, des filtres solaires, et leurs mélanges. Ces additifs peuvent être présents dans la composition en une teneur allant de 0,01 à 20 % du poids total de la composition et mieux de 0,01 à 10% (si présents).

La composition selon l'invention peut se présenter sous la forme d'émulsion huile-dans-eau, eau-dans-huile, de dispersion cire-dans-eau ou bien encore être une composition anhydre.

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

La composition selon l'invention peut être fabriquée par les procédés connus, généralement utilisés dans le domaine cosmétique ou dermatologique.

En particulier, la composition selon l'invention ne contient pas :

a) 41 % d'eau, 10 % d'isododécane, 5 % d'éthanol, 4 % d'hectorite, 10 % d'oxyde de fer noir ;
ou
b) 62,9 % d'eau, 10 % d'acide stéarique, 3 % de triéthanolamine, 4 % d'oxyde de zinc, 10 % d'oxyde de fer noir,
les pourcentages étant exprimés en poids par rapport au poids total de la composition.

L'invention est illustrée plus en détail dans les exemples suivants.

## Méthode de mesure de la masse volumique de particules solides :

La masse volumique apparente de particules solides est mesurée à l'aide d'un pycnomètre Gay-Lussac.

On utilise une balance de précision (précision de 1 mg) et les mesures sont effectuées dans une enceinte thermostatée à 25 °C ($\pm$ 0.5°C). On utilise également deux liquides de référence ayant une masse volumique Mv qui sont l'eau déminéralisée (MV = 1000 kg/m$^3$) et l'heptane (MV = 683.7 kg/m$^3$). On effectue la mesure de la masse volumique des particules solides avec chaque liquide de référence.

On place le pycnomètre et les produits utilisé pour effectuer la mesure à la température de 25°C. Les masses citées ci-après sont exprimées en kilogrammes.

On mesure la masse M0 du pycnomètre, puis on remplit complètement le pycnomètre du liquide de référence employé, en évitant l'introduction de bulle d'air.
On mesure la masse M1 du pycnomètre rempli.

Puis on prépare un mélange de masse M2 du matériau dont on veut mesurer sa masse volumique Mv2 avec une masse M3 de liquide de référence. On agite le mélange puis juste à la fin de l'agitation, on remplit le pycnomètre avec ce mélange et on mesure la masse M4 du pycnomètre rempli. On détermine ainsi la masse M4 - M0 du mélange présent dans le pycnomètre.
Le pycnomètre ayant un volume de remplissage constant, on peut donc établir la relation suivante : (M1-M0) / Mv = (M2/Mv2 + M3/Mv) x (M4-M0) / (M2+M3)

Cette relation permet de calculer la valeur de la masse volumique Mv2 des particules solides, exprimée en kg/m$^3$. On détermine ainsi pour chacun des liquides de référence une valeur de la masse volumique des particules solides. Selon l'invention, la valeur la plus élevée (parmi la masse volumique mesurée avec l'eau distillée et la masse volumique mesurée avec l'heptane) est retenue comme valeur de la masse volumique pour la détermination de la fraction volumique des particules solides.

## Méthode de mesure de rétraction :

Le principe consiste à mesurer avant traitement et après traitement la longueur d'une éprouvette de stratum cornéum isolé et de déterminer le pourcentage de rétraction de l'éprouvette.

On utilise des éprouvettes de 1 cm X 0,4 cm de stratum cornéum d'épaisseur allant de 10 à 20 μm disposées sur l'extensiomètre MTT 610 commercialisé par la société DIASTRON.

L'éprouvette est placée entre 2 mâchoires puis laissée pendant 12 heures dans une atmosphère à 30 °C et 40 % d'humidité relative.

On tracte à la vitesse de 2 mm/minute l'éprouvette d'une longueur comprise entre 5 et 10 % de la longueur initiale pour déterminer la longueur $l_1$ à partir de laquelle l'éprouvette commence à exercer une force sur les mâchoires et détectée par l'appareil.

On détend ensuite l'éprouvette puis on applique sur le stratum cornéum 2 mg d'une composition aqueuse à 7 % en poids de polymère. Après évaporation totale de l'eau de la composition, on tracte l'éprouvette dans les mêmes conditions que celles décrites précédemment pour déterminer également la longueur $l_2$ pour l'éprouvette traitée.

Le pourcentage de rétraction est déterminé par le rapport: 100 X ($I_2$ - $I_1$)/$I_1$.

**Exemple 1 :**

a) On prépare une dispersion aqueuse de cire d'abeille en mélangeant à 95 °C, 40 g de cire d'abeille, 4 g de tensioactif alcool laurylique polyoxyéthyléné à 23 motifs d'oxyde d'éthylène vendu sous la dénomination « BRIJ 35 » par la société UNICHEMA et 56 g d'eau chauffée à 95 °C, sous agitation à l'aide d'un agitateur Ultraturrax, jusqu'à obtenir une dispersion aqueuse de cire ayant une taille moyenne de particules d'environ 300 nm.

b) On prépare un mascara ayant la composition suivante :

- Polyuréthane en dispersion aqueuse à 35 % de polymère
  (AVALURE UR 410 de Goodrich)      30 g MA
- Cire d'abeille      3 g
- Poudre de Nylon-12 (Orgasol 2002 d'Atochem)      5 g
- Oxyde de fer noir (Sicovit noir 85E172 de BASF)      5 g
- Hydroxyéthylcellulose
  (Cellosize QP4400M d'Amerchol)      1 g
- Propylène glycol      5 g
- Tensioactif (Brij 35)      0,3 g
- Eau      qsp      100 g

Cette composition est préparée en mélangeant 7,5 g de la dispersion de ciré décrite au point a) précédent avec la fraction aqueuse complémentaire comprenant les autres ingrédients.
On obtient un mascara dont la fraction non volatile (formée de tous les constituants sauf l'eau) contient une fraction volumique de particules solides (cire, nylon, oxyde de fer noir) égale à 28 % (par rapport au volume total de la fraction non volatile) ; la fraction volumique des particules principales au sens de la présente invention (l'oxyde de fer noir et le nylon) représente 78,6 % du volume total des particules solides.
Les cils maquillés avec ce mascara présentent un bon recourbement.

**Exemple 2 :**

On prépare un mascara ayant la composition suivante :

- Polyuréthane en dispersion aqueuse à 35 % de polymère
  (AVALURE UR 410 de Goodrich)      15 g MA
- Copolymère styrène acrylique en dispersion aqueuse à 30 % de polymère (Joncryl SCX 8082 de Johnson Polymers)*      6,5 g MA
- Oxyde de fer noir (Sicovit noir 85E172 de BASF)      2,5 g
- Hydroxyéthylcellulose
  (Cellosize QP4400M d'Amerchol)      0,5 g
- Propylène glycol      2,5 g
- Eau      qsp      100 g

On obtient un mascara dont la fraction non volatile (formée de tous les constituants sauf l'eau) contient une fraction volumique de particules solides (copolymère styrène/acrylique, oxyde de fer noir) égale à 28 % (par rapport au volume total de la fraction non volatile) ; la fraction volumique des particules principales au sens de la présente invention (l'oxyde de fer noir et le copolymère styrène/acrylique) représente 100 % du volume total des particules solides.
Les cils maquillés avec ce mascara présentent un bon recourbement.

**Exemple 3 :**

a) On prépare une dispersion de cire dure en mélangeant à 95 °C , 40 g de cire vendue sous la dénomination « PHYTOWAX Olive 18 L 57 » par la société SOPHIM, 4 g de tensioactif alcool laurylique polyoxyéthyléné à 23 motifs d'oxyde d'éthylène vendu sous la dénomination « BRIJ 35 » par la société UNICHEMA et 56 g d'eau chauffée à 95 °C, sous agitation à l'aide d'un agitateur Ultraturrax, jusqu'à obtenir une dispersion aqueuse de cire ayant une taille moyenne de particules d'environ 300 nm.

b) On prépare un mascara ayant la composition suivante :

- Polyuréthane en dispersion aqueuse à 35 % de polymère
  (AVALURE UR 410 de Goodrich)      30 g MA
- Cire (PHYTOWAX Olive 18 L 57 de la société SOPHIM)      13 g
- Oxyde de fer noir (Sicovit noir 85E172 de BASF)      5 g
- Hydroxyéthylcellulose
  (Cellosize QP4400M d'Amerchol)      1 g
- Propylène glycol      5 g
- Tensioactif (Brij 35)      1,3 g
- Eau      qsp      100 g

Cette composition est préparée en mélangeant 32,5 g de la dispersion de cire décrite au point a) précédent avec la fraction aqueuse complémentaire comprenant les autres ingrédients.
On obtient un mascara dont la fraction non vo-
* latex ayant une température de transition vitreuse Tg = 102 °C

latile (formée de tous les constituants sauf l'eau) contient une fraction volumique de particules solides (cire et oxyde de fer noir) égale à 28 % (par rapport au volume total de la fraction non volatile) ; la fraction volumique des particules principales au sens de la présente invention (l'oxyde de fer noir et la cire) représente 100 % du volume total des particules solides.

Les cils maquillés avec ce mascara présentent un bon recourbement.

**Revendications**

1. Composition de revêtement des fibres kératiniques, notamment des cils, comprenant, dans un milieu cosmétiquement acceptable comprenant au moins un solvant volatil, une fraction non volatile comprenant :

   - a) un polymère apte à adhérer sur les matières kératiniques,
   - b) des particules principales solides à 25 °C choisies parmi :

      - i) des premières particules solides comprenant un premier matériau cristallin ou semi-cristallin solide à 25 °C présentant une transition de phase de premier ordre, de fusion ou de combustion, supérieure à 100 °C,
      - ii) des deuxièmes particules solides comprenant un deuxième matériau amorphe ayant une température de transition vitreuse supérieure ou égale à 60 °C,
      - iii) des troisièmes particules solides comprenant un troisième matériau choisi parmi les cires ayant une dureté supérieure ou égale à 6,5 MPa,
      - iv) et leurs mélanges,

   - c) et éventuellement des particules solides additionnelles différentes desdites particules solides principales, les particules solides additionnelles n'étant pas aptes à coalescer à une température inférieure ou égale à 40 °C,

   les particules solides principales et, le cas échéant, les particules solides additionnelles étant présentes dans la composition en une teneur telle que la fraction volumique des particules solides principales et, le cas échéant, des particules solides additionnelles est supérieure ou égal à 1 % et inférieure à 50 % du volume total de ladite fraction non volatile, et, le cas échéant, la fraction volumique des particules solides principales est supérieure ou égal à 50 % du volume total des particules solides principales et des particules solides additionnelles.

2. Composition selon la revendication 1, **caractérisée par le fait qu'**elle comprend des premières particules solides comprenant un matériau cristallin ou semi-cristallin présente une transition de phase de premier ordre supérieure à 100 °C.

3. Composition selon la revendication 1 ou 2, **caractérisée par le fait que** le premier matériau cristallin ou semi-cristallin présente une transition de phase de premier ordre supérieure à 120 °C.

4. Composition selon l'une quelconque des revendications précédentes, **caractérisée par le fait que** le premier matériau cristallin ou semi-cristallin présente une transition de phase de premier ordre supérieure à 150 °C.

5. Composition selon l'une quelconque des revendications précédentes, **caractérisée par le fait que** le premier matériau cristallin ou semi-cristallin a une dureté Vicker supérieure ou égale à 10, de préférence allant 10 à 7500.

6. Composition selon l'une quelconque des revendications 1 à 5, **caractérisée par le fait que** le premier matériau cristallin ou semi-cristallin a une dureté Vicker supérieure ou égale à 200, de préférence allant de 200 à 7500.

7. Composition selon l'une quelconque des revendications 1 à 6, **caractérisée par le fait que** le premier matériau cristallin ou semi-cristallin a une dureté Vicker supérieure ou égale à 400, de préférence allant de 400 à 7500.

8. Composition selon l'une quelconque des revendications précédentes, **caractérisée par le fait que** le premier matériau cristallin ou semi-cristallin est choisi dans le groupe formé par la silice, le verre, le diamant, le cuivre, le nitrure de bore, les céramiques, les oxydes métalliques, les polyamides, et leurs mélanges.

9. Composition selon la revendication 8, **caractérisée par le fait que** les oxydes métalliques sont choisis parmi les oxydes de fer.

10. Composition selon l'une quelconque des revendications précédentes, **caractérisée par le fait que** les premières particules ont une taille moyenne allant de 5 nm à 50 nm, de préférence allant de 20 nm à 50 nm.

11. Composition selon l'une quelconque des revendications précédentes, **caractérisée par le fait qu'**elle comprend des deuxièmes particules solides comprenant un deuxième matériau amorphe ayant une température de transition vitreuse supérieure ou égale à 60 °C.

**12.** Composition selon la revendication précédente, **caractérisée par le fait que** le matériau amorphe des deuxièmes particules solides a une température de transition vitreuse supérieure ou égale à 80 °C.

**13.** Composition selon l'une des revendications 11 ou 12, **caractérisée par le fait que** le matériau amorphe des deuxièmes particules solides a une température de transition vitreuse supérieure ou égale à 100 °C.

**14.** Composition selon l'une quelconque des revendications 11, **caractérisée par le fait que** le matériau amorphe est un polymère.

**15.** Composition selon l'une quelconque des revendications 11 à 14, **caractérisée par le fait que** le matériau amorphe est un polymère choisi parmi les polymères radicalaires et les polycondensats.

**16.** Composition selon l'une quelconque des revendications 11 à 15, **caractérisée par le fait que** le matériau amorphe est un polymère choisi parmi les polymères d'éthylène, les polymères de propylène, les polymères acryliques, les polymères d'acrylamide, les polymères de (méth)acrylonitrile, les polycarbonates, les polyuréthanes, les polyesters, les polyamides, les polysulfones, les polysulfonamides, les carbohydrates.

**17.** Composition selon l'une quelconque des revendications 11 à 16, **caractérisée par le fait que** les deuxièmes particules ont une taille moyenne allant de 10 nm à 50 μm, et de préférence allant de 20 nm à 1 μm.

**18.** Composition selon l'une quelconque des revendications précédentes, **caractérisée par le fait qu'**elle comprend des troisièmes particules comprenant une cire ayant une dureté supérieure ou égale à 6,5 MPa.

**19.** Composition selon la revendication précédente, **caractérisée par le fait que** la cire a un point de fusion allant de 30 °C à 99 °C, et de préférence allant de 40 °C à 99 °C.

**20.** Composition selon la revendication précédente, **caractérisée par le fait que** la cire a un point de fusion supérieur ou égal à 30 °C et inférieur à 77°C, et de préférence supérieur ou égal à 30 °C et inférieur à 60 °C.

**21.** Composition selon l'une des revendications 18 à 20, **caractérisée par le fait que** la cire a une dureté allant de 6,5 MPa à 20 MPa, notamment allant de 6,5 MPa à 15 MPa, de préférence allant de 6,5 à 12 MPa.

**22.** Composition selon l'une quelconque des revendications 18 à 21, **caractérisée par le fait que** la cire a une dureté allant de 9,7 à 20 MPa, de préférence allant de 9,7 à 15 MPa, et mieux allant de 9,7 à 12 à MPa.

**23.** Composition selon l'une quelconque des revendications 18 à 22, **caractérisée par le fait que** la cire est choisie parmi la cire de Candellila, la cire de jojoba hydrogénée, la cire de sumac, la cérésine, le stéarate d'octacosanyle, le stéarate de tétracontanyle, la cire de Shellac, le fumarate de béhényle, le tétrastéarate de di-(triméthylol-1,1,1 propane), le tétrabéhénate de di-(triméthylol-1,1,1 propane), les ozokerites, la cire obtenue par hydrogénation d'huile d'olive estérifiée avec l'alcool stéarylique, les cires obtenues par hydrogénation d'huile de ricin estérifiée avec l'alcool cétylique.

**24.** Composition selon l'une quelconque des revendications 18 à 23, **caractérisée par le fait que** la cire est choisie parmi le tétrastéarate de di-(triméthylol-1,1,1 propane) et la cire d'olive obtenue par hydrogénation d'huile d'olive estérifiée avec l'alcool stéarylique.

**25.** Composition selon l'une quelconque des revendications 18 à 24, **caractérisée par le fait que** les troisièmes particules solides ont une taille moyenne allant 50 nm à 50 μm, et de préférence de 100 nm à 10 μm.

**26.** Composition selon l'une quelconque des revendications précédentes, **caractérisée par le fait que** la composition comprend les particules solides principales et les particules solides additionnelles.

**27.** Composition selon l'une quelconque des revendications précédentes, **caractérisée par le fait que** la fraction volumique des particules solides principales et, le cas échéant, des particules solides additionnelles va de 1 % à 49 % du volume total de la fraction non volatile de la composition.

**28.** Composition selon l'une quelconque des revendications précédentes, **caractérisée par le fait que** la fraction volumique des particules solides principales et, le cas échéant, des particules solides additionnelles est supérieure ou égale à 5 % et inférieure à 50 %, de préférence va de 5 % à 49 % du volume total de la fraction non volatile de la composition.

**29.** Composition selon l'une quelconque des revendications précédentes, **caractérisée par le fait que** la fraction volumique des particules solides principales et, le cas échéant, des particules solides additionnelles est supérieure ou égale à 1 % et inférieure ou égale à 40 %, de préférence va de 5 % à 40 % du volume total de la fraction non volatile de la composition.

**30.** Composition selon l'une quelconque des revendications précédentes, **caractérisée par le fait que** la fraction volumique des particules solides principales et, le cas échéant, des particules solides additionnelles est supérieure ou égale à 1 % et inférieure ou égale à 30 %, de préférence va de 10 % à 30 %, du volume total de la fraction non volatile de la composition.

**31.** Composition selon l'une quelconque des revendications 26 à 30, **caractérisée par le fait que** les particules solides principales sont présentes dans la composition en une teneur telle que la fraction volumique des particules solides principales est supérieure ou égale à 50 %, notamment va de 50 % à 99 %, du volume total des particules solides principales et des particules solides additionnelles, de préférence est supérieure ou égale à 60 %, notamment va de 60 % à 99 %, et mieux est supérieure ou égale à 70 %, notamment va de 70 % à 99 %.

**32.** Composition selon l'une quelconque des revendications précédentes, **caractérisée par le fait que** le solvant volatil est choisi parmi l'eau, les solvants organiques volatiles, les huiles volatiles, et leurs mélanges.

**33.** Composition selon l'une quelconque des revendications précédentes, **caractérisée par le fait que** le polymère apte à adhérer sur les matières kératiniques est choisi dans le groupe formé par les polymères vinyliques, les polyuréthanes, les polyesters, les polyamides, les polyurées, les polymères cellulosiques.

**34.** Composition selon l'une quelconque des revendications précédentes, **caractérisée par le fait que** le polymère apte à adhérer sur les matières kératiniques est un polymère filmogène à une température inférieure ou égale à 40 °C.

**35.** Composition selon l'une quelconque des revendications précédentes, **caractérisée par le fait que** le polymère apte à adhérer sur les matières kératiniques est un polymère apte à former un dépôt produisant à une concentration de 7 % dans l'eau, une rétraction du stratum corneum isolé de plus de 1 % à 30°C sous une humidité relative de 40 %.

**36.** Composition selon la revendication précédente, **caractérisée par le fait que** la rétraction du stratum cornéum est de plus de 1,2 %, et mieux de plus de 1,5 %.

**37.** Composition selon l'une quelconque des revendications précédentes, **caractérisée par le fait que** polymère apte à adhérer sur les matières kératiniques est présent en une teneur allant de 0,1 % à 50 % en poids, par rapport au poids total de la composition, de préférence de 0,5 % à 40 % en poids, et mieux de 1 % à 30 % en poids.

**38.** Composition selon l'une quelconque des revendications précédentes, **caractérisée par le fait qu'**elle comprend une phase aqueuse.

**39.** Composition selon l'une quelconque des revendications précédentes, **caractérisée par le fait qu'**elle comprend une phase aqueuse formée d'eau ou d'un mélange d'eau et de solvant organique miscible à l'eau.

**40.** Composition selon la revendication précédente, **caractérisée par le fait que** le solvant organique miscible à l'eau est choisi parmi les monoalcools inférieurs ayant de 1 à 5 atomes de carbone, les glycols ayant de 2 à 8 atomes de carbone, les cétones en $C_3$-$C_4$, les aldéhydes en $C_2$-$C_4$.

**41.** Composition selon l'une des revendications 39 ou 40, **caractérisée par le fait que** la phase aqueuse est présente en une teneur allant de 0,1 % à 98 % en poids, par rapport au poids total de la composition, de préférence allant de 1 % à 80 % en poids, et mieux allant de 5 % à 65 % en poids.

**42.** Composition selon l'une quelconque des revendications 39 à 41, **caractérisée par le fait que** le polymère apte à adhérer sur les matières kératiniques est solubilisé dans la phase aqueuse.

**43.** Composition selon l'une quelconque des revendications 39 à 41, **caractérisée par le fait que** le polymère apte à adhérer sur les matières kératiniques est sous forme de particules solides en dispersion aqueuse.

**44.** Composition selon l'une quelconque des revendications précédentes, **caractérisée par le fait qu'**elle comprend une huile volatile.

**45.** Composition selon la revendication précédente, **caractérisée par le fait que** l'huile volatile est choisies parmi les huiles hydrocarbonées, les huiles siliconées, les huiles fluorées, ou leurs mélanges.

**46.** Composition selon l'une des revendications 44 ou 45, **caractérisée par le fait que** l'huile volatile est présente en une teneur allant de 0,1 % à 98 % en poids, par rapport au poids total de la composition, de préférence allant de 1 % à 65 % en poids, et mieux allant de 5 % à 65 % en poids.

**47.** Composition selon l'une quelconque des revendications précédentes, **caractérisée par le fait qu'**elle comprend une huile non volatile.

**48.** Composition selon la revendication précédente, **caractérisée par le fait que** l'huile non volatile est présente en une teneur allant de 0,1 % à 50 % en poids, de préférence de 0,1 % à 30 % en poids, par rapport au poids total de la composition, et mieux de 0,1 % à 20 % en poids.

**49.** Composition selon l'une quelconque des revendications précédentes, **caractérisée par le fait que** le polymère apte à adhérer sur les matières kératiniques est solubilisé ou dispersé sous forme de particules stabilisées en surface dans une phase grasse liquide.

**50.** Composition selon l'une quelconque des revendications précédentes, **caractérisée par le fait qu'**elle comprend une cire additionnelle différente de la cire des troisièmes particules solides.

**51.** Composition selon la revendication précédente , **caractérisée par le fait que** la cire additionnelle est présente dans la composition en une teneur allant de 0,1 % à 35 % en poids, par rapport au poids total de la composition, de préférence de 0,1 % à 20 % en poids, et mieux de 1 % à 10 % en poids.

**52.** Composition selon l'une la revendication 50 ou 51, **caractérisée par le fait que** la cire additionnelle est sous forme de particules ayant une taille moyenne allant de 50 nm à 50 μm, et de préférence de 50 nm à 10 μm.

**53.** Composition selon l'une quelconque des revendications précédentes, **caractérisée par le fait qu'**elle comprend un tensioactif.

**54.** Composition selon l'une quelconque des revendications précédentes, **caractérisée par le fait qu'** elle comprend un additif choisi parmi les matières colorantes, les antioxydants, les charges, les conservateurs, les parfums, les neutralisants, les épaississants, les actifs cosmétiques, les filtres solaires, les agents de coalescence, les plastifiants.

**55.** Composition selon l'une quelconque des revendications précédentes, **caractérisée par le fait qu'**elle est un mascara.

**56.** Composition selon l'une quelconque des revendications précédentes, **caractérisée par le fait qu'**elle ne contient pas :

> a) 41 % d'eau, 10 % d'isododécane, 5 % d'éthanol, 4 % d'hectorite, 10 % d'oxyde de fer noir ; ou
> b) 62,9 % d'eau, 10 % d'acide stéarique, 3 % de triéthanolamine, 4 % d'oxyde de zinc, 10 % d'oxyde de fer noir,
> les pourcentages étant exprimés en poids par rapport au poids total de la composition.

**57.** Procédé de maquillage ou de soin non thérapeutique des fibres kératiniques, notamment des cils, comprenant l'application sur les fibres kératiniques d'une composition selon l'une quelconque des revendications 1 à 56.

**58.** Utilisation d'une composition de revêtement des fibres kératiniques selon l'une quelconque des revendications 1 à 56, pour recourber les fibres kératiniques, notamment les cils.

**59.** Utilisation de particules principales solides à 25 °C choisies parmi :

> - i) des premières particules solides comprenant un premier matériau cristallin ou semi-cristallin solide à 25 °C présentant une transition de phase de premier ordre, de fusion ou de combustion, supérieure à 100 °C,
> - ii) des deuxièmes particules solides comprenant un deuxième matériau amorphe ayant une température de transition vitreuse supérieure ou égale à 60 °C,
> - iii) des troisièmes particules solides comprenant un troisième matériau choisi parmi les cires ayant une dureté supérieure ou égale à 6,5 MPa,
> - iv) et leurs mélanges,

et éventuellement de particules solides additionnelles différentes desdites particules solides principales, les particules solides additionnelles n'étant pas aptes à coalescer à une température inférieure ou égale à 40 °C,
dans une composition de revêtement des fibres kératiniques, notamment un mascara, comprenant, dans un milieu cosmétiquement acceptable comprenant au moins un solvant volatil, une fraction non volatile comprenant un polymère apte à adhérer sur les matières kératiniques, lesdites particules solides principale et, éventuellement, lesdites particules solides additionnelles, les particules solides principales et, le cas échéant, les particules solides additionnelles étant présentes dans la composition en une teneur telle que la fraction volumique des particules solides principales et, le cas échéant, des particules solides additionnelles est supérieure ou égal à 1 % et inférieure à 50 % du volume total de ladite fraction non volatile,
et, le cas échéant, la fraction volumique des particules solides principales est supérieure ou égal à 50 % du volume total des particules solides principales et des particules solides additionnelles,
pour recourber les fibres kératiniques, notamment les cils.

Office européen des brevets

## RAPPORT DE RECHERCHE EUROPEENNE

Numéro de la demande

EP 02 29 1694

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | EP 1 108 415 A (L'ORÉAL) 20 juin 2001 (2001-06-20) * page 7, ligne 5 - ligne 20; revendications 1-47; exemples 1-4 * | 1-59 | A61K7/032 |
| A | EP 1 064 920 A (L'ORÉAL) 3 janvier 2001 (2001-01-03) * page 14, ligne 14 - ligne 52; revendications 1-36; exemples 1,2 * | 1-59 | |
| A | FR 2 794 970 A (L'ORÉAL) 22 décembre 2000 (2000-12-22) * page 14, ligne 32 - page 15, ligne 30; revendications 1-18; exemples 3-5 * | 1-59 | |
| A | EP 1 082 953 A (L'ORÉAL) 14 mars 2001 (2001-03-14) * revendications 1-25; exemples 1-22 * | 1-59 | |
| A | EP 0 557 196 A (L'ORÉAL) 25 août 1993 (1993-08-25) * revendications 1-19; exemples 15-20 * | 1-59 | DOMAINES TECHNIQUES RECHERCHES (Int.Cl.7) A61K |
| A | EP 1 048 282 A (L'ORÉAL) 2 novembre 2000 (2000-11-02) * revendications 1-21; exemple 1 * | 1-59 | |
| P,X | EP 1 201 222 A (L'ORÉAL) 2 mai 2002 (2002-05-02) * colonne 12, ligne 53 - colonne 13, ligne 13; revendications 1-39; exemples 1,2 * | 1-59 | |
| A | EP 1 040 814 A (L'ORÉAL) 4 octobre 2000 (2000-10-04) * exemples 1,2 * | 1 | |

-/--

Le présent rapport a été établi pour toutes les revendications

| Lieu de la recherche | Date d'achèvement de la recherche | Examinateur |
|---|---|---|
| LA HAYE | 12 décembre 2002 | Willekens, G |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

# EP 1 281 385 A1

**Office européen des brevets**

## RAPPORT DE RECHERCHE EUROPEENNE

Numéro de la demande

EP 02 29 1694

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | US 6 001 168 A (V. HALL-GOULLE ET AL.)<br>14 décembre 1999 (1999-12-14)<br>* exemples 2-6 * | 1 | |
| | | | **DOMAINES TECHNIQUES RECHERCHES (Int.Cl.7)** |

Le présent rapport a été établi pour toutes les revendications

| Lieu de la recherche | Date d'achèvement de la recherche | Examinateur |
|---|---|---|
| LA HAYE | 12 décembre 2002 | Willekens, G |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE**
**RELATIF A LA DEMANDE DE BREVET EUROPEEN NO.**            EP 02 29 1694

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

12-12-2002

| Document brevet cité au rapport de recherche | | | Date de publication | Membre(s) de la famille de brevet(s) | | | Date de publication |
|---|---|---|---|---|---|---|---|
| EP | 1108415 | A | 20-06-2001 | FR | 2801501 | A1 | 01-06-2001 |
| | | | | CN | 1306812 | A | 08-08-2001 |
| | | | | EP | 1108415 | A2 | 20-06-2001 |
| | | | | JP | 2001192559 | A | 17-07-2001 |
| | | | | US | 2001006665 | A1 | 05-07-2001 |
| EP | 1064920 | A | 03-01-2001 | FR | 2795634 | A1 | 05-01-2001 |
| | | | | BR | 0006944 | A | 31-07-2001 |
| | | | | CA | 2341591 | A1 | 11-01-2001 |
| | | | | CN | 1321079 | T | 07-11-2001 |
| | | | | EP | 1064920 | A1 | 03-01-2001 |
| | | | | WO | 0101936 | A1 | 11-01-2001 |
| | | | | JP | 2001031539 | A | 06-02-2001 |
| | | | | US | 6482400 | B1 | 19-11-2002 |
| FR | 2794970 | A | 22-12-2000 | FR | 2794970 | A1 | 22-12-2000 |
| | | | | EP | 1066819 | A1 | 10-01-2001 |
| | | | | JP | 2001039830 | A | 13-02-2001 |
| EP | 1082953 | A | 14-03-2001 | FR | 2798061 | A1 | 09-03-2001 |
| | | | | EP | 1082953 | A1 | 14-03-2001 |
| | | | | JP | 2001089325 | A | 03-04-2001 |
| EP | 557196 | A | 25-08-1993 | FR | 2687569 | A1 | 27-08-1993 |
| | | | | AT | 132360 | T | 15-01-1996 |
| | | | | CA | 2089984 | A1 | 22-08-1993 |
| | | | | DE | 69301166 | D1 | 15-02-1996 |
| | | | | DE | 69301166 | T2 | 29-08-1996 |
| | | | | EP | 0557196 | A1 | 25-08-1993 |
| | | | | ES | 2082598 | T3 | 16-03-1996 |
| | | | | JP | 6009341 | A | 18-01-1994 |
| | | | | US | 5849278 | A | 15-12-1998 |
| | | | | US | 5858338 | A | 12-01-1999 |
| EP | 1048282 | A | 02-11-2000 | FR | 2792829 | A1 | 03-11-2000 |
| | | | | BR | 0001231 | A | 02-05-2001 |
| | | | | CA | 2307085 | A1 | 28-10-2000 |
| | | | | CN | 1273083 | A | 15-11-2000 |
| | | | | EP | 1048282 | A1 | 02-11-2000 |
| | | | | JP | 2000319127 | A | 21-11-2000 |
| EP | 1201222 | A | 02-05-2002 | FR | 2815850 | A1 | 03-05-2002 |
| | | | | BR | 0105675 | A | 25-06-2002 |
| | | | | CN | 1350835 | A | 29-05-2002 |
| | | | | EP | 1201222 | A1 | 02-05-2002 |
| | | | | JP | 2002179532 | A | 26-06-2002 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE**
**RELATIF A LA DEMANDE DE BREVET EUROPEEN NO.**    EP 02 29 1694

La présente annexe indique les membres de la famille de brevets relatifs aux documents brevets cités dans le rapport de
recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

12-12-2002

| Document brevet cité au rapport de recherche | | Date de publication | Membre(s) de la famille de brevet(s) | | Date de publication |
|---|---|---|---|---|---|
| EP 1201222 | A | | US | 2002085986 A1 | 04-07-2002 |
| EP 1040814 | A | 04-10-2000 | FR | 2791561 A1 | 06-10-2000 |
| | | | AT | 215350 T | 15-04-2002 |
| | | | BR | 0001105 A | 24-04-2001 |
| | | | CA | 2302854 A1 | 01-10-2000 |
| | | | CN | 1279058 A | 10-01-2001 |
| | | | DE | 60000103 D1 | 08-05-2002 |
| | | | DE | 60000103 T2 | 14-08-2002 |
| | | | EP | 1040814 A1 | 04-10-2000 |
| | | | ES | 2174801 T3 | 16-11-2002 |
| | | | JP | 2000290141 A | 17-10-2000 |
| | | | US | 6372201 B1 | 16-04-2002 |
| US 6001168 | A | 14-12-1999 | AU | 7211498 A | 30-10-1998 |
| | | | AU | 8541098 A | 25-01-1999 |
| | | | AU | 8730198 A | 25-01-1999 |
| | | | CN | 1252135 T | 03-05-2000 |
| | | | CN | 1261908 T | 02-08-2000 |
| | | | DE | 69807203 D1 | 19-09-2002 |
| | | | WO | 9845757 A1 | 15-10-1998 |
| | | | WO | 9901511 A1 | 14-01-1999 |
| | | | WO | 9901512 A1 | 14-01-1999 |
| | | | EP | 0974075 A1 | 26-01-2000 |
| | | | EP | 0993489 A1 | 19-04-2000 |
| | | | EP | 0993490 A1 | 19-04-2000 |
| | | | JP | 2002514263 T | 14-05-2002 |
| | | | JP | 2002508802 T | 19-03-2002 |
| | | | JP | 2002512647 T | 23-04-2002 |
| | | | US | 6010567 A | 04-01-2000 |
| | | | US | 6071989 A | 06-06-2000 |
| | | | US | 6165681 A | 26-12-2000 |
| | | | US | 6211347 B1 | 03-04-2001 |

EPO FORM P0460

Pour tout renseignement concernant cette annexe : voir Journal Officiel de l'Office européen des brevets, No. 12/82